# EUROPEAN PATENT SPECIFICATION

(11) **EP 2 540 307 B1**
(45) Date of publication and mention of the grant of the patent: **25.05.2016**
(21) Application number: 11747391.8
(22) Date of filing: 23.02.2011
(51) Int. Cl.: A61K 36/02, A23L 2/44, A23L 3/34, A61K 31/727, A61K 38/44, A61P 31/04

(54) **ANTIBACTERIAL AUXILIARY AGENT COMPRISING KOMBU EXTRACT AS ACTIVE INGREDIENT, ANTIBACTERIAL COMPOSITION, AND FOOD OR BEVERAGE**
ANTIBAKTERIELLES HILFSMITTEL MIT EINEM KOMBU-EXTRAKT ALS WIRKSTOFF, ANTIBAKTERIELLE ZUSAMMENSETZUNG UND NAHRUNGSMITTEL ODER GETRÄNK DAMIT
AGENT AUXILIAIRE ANTIBACTÉRIEN COMPRENANT UN EXTRAIT DE KONBU AU TITRE DE PRINCIPE ACTIF, COMPOSITION ANTIBACTÉRIENNE ET ALIMENT OU BOISSON

(30) Priority: 24.02.2010 JP 2010039218
(43) Date of publication of application: 02.01.2013
(73) Proprietor: Morinaga Milk Industry Co., Ltd., Minato-ku Tokyo 108-8384 (JP)
(72) Inventor: SHIN,Kouichirou, Zama-shi Kanagawa 252-8583 (JP); YAMAUCHI,Koji, Zama-shi Kanagawa 252-8583 (JP)
(74) Representative: Adam, Holger
(86) International application number: PCT/JP2011/053996
(87) International publication number: WO 2011/105431

(56) References cited:
- JP-A- 9 048 715
- JP-A- 11 180 813
- JP-A- 2000 509 367
- JP-A- 2001 240 604
- JP-A- 2002 544 212
- US-A1- 2006 210 609
- SEUNG-BEOM PARK ET AL: "The differential effect of high and low molecular weight fucoidans on the severity of collagen-induced arthritis in mice", PHYTOTHERAPY RESEARCH, vol. 24, no. 9, 1 September 2010 (2010-09-01), pages 1384-1391, XP055066986, ISSN: 0951-418X, DOI: 10.1002/ptr.3140
- LI L -Y ET AL: "Evaluation of in vitro antioxidant and antibacterial activities of Laminaria japonica polysaccharides", JOURNAL OF MEDICINAL PLANT RESEARCH 2010 ACADEMIC JOURNALS NGA, vol. 4, no. 21, November 2010 (2010-11), pages 2194-2198, XP009170428, ISSN: 1996-0875
- GUPTA SHILPI ET AL: "Study of the microbial diversity and antimicrobial properties of Irish edible brown seaweeds", INTERNATIONAL JOURNAL OF FOOD SCIENCE & TECHNOLOGY, vol. 45, no. 3, March 2010 (2010-03), pages 482-489, XP002698996, ISSN: 0950-5423
- KOUICHIROU SHIN ET AL: "Antibacterial Activity of the Lactoperoxidase System Combined with Edible Laminaria Hot-Water Extract as a Source of Halide Ions", BIOSCIENCE, BIOTECHNOLOGY, AND BIOCHEMISTRY, vol. 76, no. 2, 1 January 2012 (2012-01-01), pages 404-406, XP055067053, ISSN: 0916-8451, DOI: 10.1271/bbb.110735
- KOICHIRO SHIN: 'Konbu Chushutsubutsu o Riyo shita Lactoperoxidase System no Kokin Kassei' NIPPON NOGEI KAGAKUKAI TAIKAI KOEN YOSHISHU vol. 186, 05 March 2010, XP008169672

## Description

### TECHNICAL FIELD

The present invention relates to an antibacterial supplement comprising a fraction having a molecular weight of 5000 or less obtained from a kombu (kelp) extract(s) for use in an antibacterial composition with lactoperoxidase system for securing the sanitary conditions in the body.

Further, the present invention relates to an antibacterial composition for use in securing the sanitary conditions in a body comprising the antibacterial supplement comprising a fraction having a molecular weight of 5000 or less obtained from a kombu extract,, lactoperoxidase, glucose oxidase and glucose.

Further, the present invention relates to a food and beverage (drink) containing said antibacterial composition for use in securing the sanitary conditions in a body.

### BACKGROUND ART

Kombu is an inevitable one for Japanese eating habits, and has been taken habitually for long term as foodstuff rich in nutrition such as minerals.

In general, kombu is a generic name of marine plants of Phaeophyceae, Laminariaceae laminaria and related genus. As an eatable kombu, makombu (nomenclature: Laminaria japonica), rausukombu (nomenclature: Laminaria diabolica), rishirikombu (nomenclature: Laminaria ochotensis), hidakakombu (nomenclature: Laminaria angustata), and the like are known.

Kombu soup stock which is a hot water extract of kombu includes a flavoring substrate consisting mainly of glutamine, and food fiber consisting mainly of alginic acid, and in addition to them, abundant minerals such as potassium, sodium, calcium, magnesium, iron and the like.

Preparation methods for preparing a kombu extract in which a particular ingredient among the ones described above is increased or decreased have been disclosed (e.g., Patent Document 1 and Patent Document 2). Up to this time, the properties of the flavoring substrate (taste component) and the nutritional value of food fiber and minerals have been mainly utilized. In addition, several physiological effects of kombu extracts such as anticancer effect (e.g., Non-Patent Document 1), antioxidant effect (e.g., Non-Patent Document 2), anti-mutagenic effect (e.g., Non-Patent Document 3) and the like have been disclosed.

Further, an antibacterial agent containing the residue of extracting the seaweed ash as an active ingredient is disclosed (e.g., Patent Document 3). Further, an antibacterial composition for E. coli which is characterized by including hot water extract of a seaweed of the family Spermatochnaceae, considered a delicacy when seasoned with vinegar and/or the processed one (e.g., Patent Document 4).

In addition, an oral composition containing the seaweed extract as an active ingredient (e.g., Patent Document 5). Further, a composition for the oral cavity for the prophylaxis and therapy periodontal diseases obtained by using a partially hydrolyzed polysaccharide extracted from seaweed (e.g., Patent Document 6).

However, Patent Document 3 is directed to the residue of extracting the raw material marine algae. Patent Document 4 does not disclose the kombu, and it does not disclose any microbial other than the pathogenic E. coli O-157.

Patent Document 5 does not disclose the ingredients of the extract of marine algae, and the objective microbial which exhibits antibacterial effect is defined to Porphyromonas gingivalis.

Patent Document 6 describes that in order to obtain polysaccharide, it is required to extract and partially hydrolyze the marine algae. Further, Patent Documents 5 and 6 describe that as an extraction solvent, a chloroform-methanol mixed solvent is preferably used which is not suit for food.

On the other hand, lactoperoxidase, one of milk protein is an oxidoreductase included in the secretion fluid such as milk, saliva, tear fluid, airway mucus and the like of mammalians, which can be purified industrially from cow milk in large scale.

It is disclosed that lactoperoxidase increases the livability of Bifidobacterium and Lactobacillus (e.g., Patent Document 7). Further, a system where lactoperoxidase catalyzes the production of hypothiocyanate in the presence of hydrogen peroxide and thiocyanate exhibits a potent antibacterial activity is known. These are known as a lactoperoxidase system.

Among the combination, thiocyanate is not approved as a food additive, and thus, it could not be directly used for food and drink.

### PRIOR ART DOCUMENTS

### PATENT DOCUMENTS

PATENT DOCUMENT 1:
   Japanese Patent Application Public Disclosure (Laid-Open) No. 2000-270806
PATENT DOCUMENT 2:
   Japanese Patent Application Publication (Publication of Provisionally Allowed Application) No. Hei 05-77379
PATENT DOCUMENT 3:
   Japanese Patent Application Public Disclosure (Laid-Open) No. 2000-344677
PATENT DOCUMENT 4:
   Japanese Patent Application Public Disclosure (Laid-Open) No. 2000-344679
PATENT DOCUMENT 5:
   Japanese Patent Application Public Disclosure (Laid-Open) No. Hei 09-48715
PATENT DOCUMENT 6:
   Japanese Patent Application Public Disclosure (Laid-Open) No. 2001-240604
PATENT DOCUMENT 7:
   Japanese Patent Application Public Disclosure (Laid-Open) No. Hei 05-41981

### NON-PATENT DOCUMENTS

NON-PATENT DOCUMENT 1:
   Cancer Letters, Ireland, Vol. 30, 1986, p. 125-131
NON-PATENT DOCUMENT 2:
   Biological and Pharmaceutical Bulletin, Japan, Vol. 27, 2004, p. 1037-1040
NON-PATENT DOCUMENT 3:
   Mutation Research, Holland, Vol. 303, 1993, p. 63-70

### OUTLINE OF INVENTION

### PROBLEM TO BE SOLVED BY THE INVENTION

The inventors of the present invention have eagerly sought a novel use application of kombu repeatedly in order to utilize the physiological activity of kombu, considering the above-described BACKGROUND ART, and thus, found that among the kombu extract (extract of kombu), a fraction having a molecular weight of 5000 or less can be used as an antibacterial supplement.

Further, it was found that said antibacterial supplement exhibits an action as a antibacterial composition having a potent antibacterial activity when said antibacterial supplement is used together with lactoperoxidase, glucose oxidase and glucose.

Further, it was found that said antibacterial composition can be preferably used for food and drink.

### MEANS FOR SOLVING PROBLEM

The first present invention for solving said problem is an antibacterial supplement including a fraction having a molecular weight of 5000 or less obtained from kombu extract (extract of kombu).

The first present invention is for use in an antibacterial supplement in the antibacterial composition with a lactoperoxidase system for securing the sanitary conditions in a body.

The first present invention is preferably an invention wherein the kombu extract is an extract of one or more of kombu selected from the group consisting of makombu (Laminaria japonica), rausu kombu (Laminaria diabolica), rishiri kombu (Laminaria ochotensis) and hidaka kombu (Laminaria angustata).

The antibacterial supplement of the first present invention is preferably used as an antibacterial supplement included in an antibacterial composition for Staphylococcus aureus.

Further, the second present invention is an antibacterial composition for use as defined above comprising the antibacterial supplement and lactoperoxidase, glucose oxidase and glucose.

The third present invention is an antibacterial for use as defined above composition comprising an additional xanthan gum. The antibacterial composition of the third present invention is preferably used as a thickener.

The fourth present invention is foods and drinks including the antibacterial composition of the present invention.

Further, the present disclosure also comprises the following (1) to (2).

Pharmaceutical composition for enhancing antibacterial activity, including, as an active ingredient, a fraction having molecular weight of 5000 or less obtained from kombu extract.

An agent for enhancing antibacterial activity, including as an active ingredient a fraction having molecular weight of 5000 or less obtained from kombu extract.

An additive for enhancing antibacterial activity, including as an active ingredient a fraction having molecular weight of 5000 or less obtained from kombu extract.

Further, the present invention also comprises the following (4) to (7).

An additive for use in an antibacterial composition with lactoperoxidase system, including a fraction having molecular weight of 5000 or less obtained from kombu extract.

A method for preparing an additive for lactoperoxidase system comprising obtaining kombu extract by performing the extraction from the kombu with a solvent(s), fractionating the kombu extract into a fraction having a molecular weight of 5000 or less.

The method according to (5), wherein the solvent is water or aqueous solution.

An antibacterial composition for use in securing the sanitary conditions including a fraction having a molecular weight of 5000 or less obtained from the kombu extract, lactoperoxidase, glucose oxidase and glucose. Further, the present invention comprises the following (8) to (10).

A method for preparing an antibacterial composition including as an active ingredient a fraction having a molecular weight of 5000 or less obtained from the kombu extract, lactoperoxidase, glucose oxidase and glucose, comprising obtaining kombu extract by performing the extraction from the kombu with a solvent(s), fractionating the kombu extract into a fraction having a molecular weight of 5000 or less, mixing the fractionated fraction having a molecular weight of 5000 or less, lactoperoxidase, glucose oxidase and glucose.

The method according to (8), wherein the solvent is water or aqueous solution. (10)

The antibacterial composition for use according to (7), wherein thiocyanate and salt thereof are not included.

Further, the present disclosure also comprises use of the fraction having a molecular weight of 5000 or less fractionated from the kombu extract (extract of kombu) for preparing said antibacterial composition. Further, the present disclosure also comprises a method for sterilizing microorganisms, mundifying, and destroying bacteria by administering said antibacterial composition. Further, the present disclosure also comprises use of said antibacterial composition for sterilizing microorganisms, mundifying, and destroying bacteria. The additive for lactoperoxidase system may be also called as an additive for antibacterial, an additive for antibacterial composition, an antibacterial supplement, an agent for enhancing antibacterial activity, a composition for enhancing antibacterial activity. In a preferred embodiment, the antibacterial composition of the present invention is one containing xanthan gum.

Further, the present disclosure also comprises the following (11) to (15).
(11) An antibacterial supplement, including, the fraction having a molecular weight of 5000 or less obtained from the kombu extract, wherein the antibacterial supplement is used in the antibacterial composition with lactoperoxidase system.
(12) The antibacterial supplement according to (11), wherein the kombu extract is an extract of one or more of kombu selected from the group consisting of makombu (Laminaria japonica), rausu kombu (Laminaria diabolica), rishiri kombu (Laminaria ochotensis) and hidaka kombu (Laminaria angustata).
(13) An antibacterial composition for use in securing the sanitary conditions in a body comprising the antibacterial supplement according to any one of (11) to (12) and lactoperoxidase, glucose oxidase and glucose.
(14) The antibacterial composition for use according to (13), further including xanthan gum.
(15) Foods and drinks containing the antibacterial composition for use according to (13) or (14).

### EFFECT OF THE INVENTION

The antibacterial supplement and antibacterial composition for use according to the present invention are used for securing the sanitary conditions in a body, especially in an oral cavity. Further, they are safe and have little side effect, and thus, they can be taken together with foods and drinks. The antibacterial supplement and antibacterial composition for use according to the present invention can be taken routinely for long term without any anxiety.

Further, the antibacterial supplement and antibacterial composition for use according to the present invention can be disseminated to places in our living environment where bacteria can be increased easily.

The antibacterial supplement according to the present invention can achieve the following effects.
(1) The antibacterial supplement according to the present invention can achieve antibacterial effect safely by taking it / by using it in an antibacterial composition with lactoperoxidase system (antibacterial composition for use according to the present invention).
(2) Lactoperoxidase system antibacterial composition using the antibacterial supplement according to the present invention has an effect on the prophylaxis (prevention) and/or treatment of diseases caused by bacteria.
(3) Lactoperoxidase system antibacterial composition using the antibacterial supplement according to the present invention has a high safety to human being and thus can be taken routinely (daily).
(4) The antibacterial composition for use according to the present invention exerts antibacterial effect by lactoperoxidase system safely.
(5) The antibacterial supplement has an effect on the prophylaxis and/or treatment of diseases caused by bacteria.
(6) The antibacterial supplement has a high safety to human being and thus can be taken routinely.
(7) Foods and drinks having an antibacterial effect can be easily provided by adding the antibacterial auxiliary to them.
(8) The antibacterial composition including xanthan gum has an effect as a thickener, and therefore, it can be provided as the foods and drinks having a low risk of bacterial infection to the person with swallowing difficulty (dysphagia) and aged person, as it is or by adding into foods and drinks.

Solving the present problem, the antibacterial supplement and antibacterial composition comprising the kombu extract can be used safely.

### MODES FOR CARRYING OUT THE INVENTION

Then, the preferred embodiments of the present invention are explained in detail.. However, the present invention is not limited within the following preferred embodiments. It can be modified freely within the scope of the present invention. In the present invention, percentage is expressed by mass unless otherwise indicated.

### [Kombu Extract]

Kombu extract which is used in the present invention can be prepared from the commercially obtainable kombu by using a standard method.

As the kombu which is used as a starting raw material for obtaining the kombu extract (extract of kombu) according to the present invention, makombu (Laminaria japonica), rausukombu (Laminaria diabolica), rishirikombu (Laminaria ochotensis), hidakakombu (Laminaria angustata), nagakombu (Laminaria longissima), hosomekombu (Laminaria religiosa), gatsugarakombu (Laminaria coriacea), chijimikombu (Laminaria cichorioides), goheikombu (Laminaria yezoensis) and the like are preferable. Especially, makombu, rausukombu, rishirikombu and hidakakombu are preferable. These can be used alone or together.

Kombu in the form for ordinary use as food such as raw kombu, freeze-dried kombu or dried kombu can be used. Dried kombu is preferably used.

As for the preparation method of the kombu extract, for example, dried kombu is soaked in water and then extracted at 80 to 100°C for 5 to 60 minutes, and thus, the kombu extract can be obtained.

As for the solvent for extracting the kombu, in addition to water, organic solvents can be used. As for the organic solvent, for example, chloroform, methanol, ethanol, propanol, dimethyl sulfoxide, hexane and the like can be used.

Among these solvents for extraction, water is preferably used because it can be used safely and easily.

As for the kombu extract, a commercially available kombu extract in the form of fluid or powder (for example, ones sold by San-Ei Gen F. F. I., Inc. and the like) also can be used.

Antibacterial supplement according to the present invention (an additive for lactoperoxidase system) can be prepared by a preparation method comprising a step wherein the kombu extract is obtained by performing the extraction of kombu with a solvent(s), and a step wherein the kombu extract is fractionated into a fraction having a molecular weight of 5000 or less.

As for the solvent used for extraction, the above-mentioned solvents can be used. In preferred embodiment, the solvent is water or an aqueous solution. As for the aqueous solution, any aqueous solution can be used with no limitation when it can be used for extraction in the same way as water and taken by human being safely. As for the aqueous solution, for example, solutions containing sodium chloride, potassium chloride, dextrin and the like can be exemplified. Extraction by solvent can be performed at ambient or warmed temperature by soaking in the solvent. In preferred embodiment, extraction by solvent can be performed by soaking in water at ambient temperature, and then warming. As for warming, it can be performed generally by warming up to the temperature of 50 to 100°C, preferably 60 to 100°C, more preferably 70 to 100°C, even more preferably 80 to 100°C, even more preferably 90 to 100°C. Duration time of extraction can be set properly. In preferred embodiment, generally, the extraction can be performed for 10 to 120 minutes, preferably 20 to 100 minutes, more preferably 30 to 90 minutes, even more preferably 40 to 80 minutes, and even more preferably 50 to 60 minutes.

As for fractionation, any means by which the fraction having the molecular weight of 5000 or less can be fractionated from the extract of kombu can be used. As for means for fractionation, filtration with a filtration membrane, filtration with a filter, filtration with a hollow fiber, centrifugation, molecular weight chromatography (gel filtration) and the like can be mentioned. Further, as such filtration, for example, dialysis, centrifugal filtration, suction filtration, pressure filtration, ultrafiltration and the like can be mentioned. Those skilled in the art can fractionate the fraction having molecular weight of 5000 or less, or 4000 or less, by selecting the filter, pore size of filter, or acceleration for centrifugation.

Kombu has been used as food and drink historically. Especially, in Japan, hot water extract of kombu is used as traditional seasoning called "kombu-dashi". Therefore, a very high level safety of extract of kombu to human being has been secured.

On the other hand, thiocyanate and salts thereof are chemical compounds which have not yet approved to add to foods in several countries.

The present invention, where thiocyanate and salts thereof need not be added, makes it possible to exert the antibacterial action of lactoperoxidase system by using only the ingredient(s) which is (are) very safe, secure and reliable. The present invention achieves a wide application of lactoperoxidase system to human being.

Extract of kombu had not been known to be able to use as an additive (antibacterial supplement) for lactoperoxidase system. As shown in the working example of the present application, the extract of kombu does not exert the effect as the additive for lactoperoxidase system as it is. However, as shown in the present invention, the present inventors found that the fraction having molecular weight of 5000 or less fractionated from the extract of kombu exerts as an additive for lactoperoxidase system though the mechanism is not clear, and thus, achieved the present invention.

The antibacterial supplement according to the present invention includes the fraction having molecular weight of 5000 or less fractionated from the extract of kombu. Therefore, in order to use as the antibacterial supplement, it is preferable to eliminate the ingredients having molecular weight greater than 5000 from the kombu extract.

Further, the antibacterial supplement according to the present invention also includes the fraction having molecular weight of 4000 or less. Therefore, in order to be able to use as the antibacterial supplement according to the present invention, it is more preferable to eliminate the ingredients having molecular weight greater than 4000 from the kombu extract.

The method for fractionating the extract of kombu is not particularly limited. However, it is preferable to use a ultrafiltration membrane (UF membrane), nano-filtration membrane (NF membrane), gel filtration and the like. Using ultrafiltration is more preferable because it is easy.

### [Lactoperoxidase]

Lactoperoxidase used for the present invention can be obtained from the milk of mammalians and the like. It can be obtained from the milk and the like of human being, bovine, horse, sheep, goat and the like. For example, as the method disclosed in Japanese Patent Application Public Disclosure No. Hei 05-41981, it is preferable to prepare the peroxidase industriously according to the standard method (e.g., ion exchange chromatography and the like) from the unheated whey or skim milk such as milk. It is preferable to use a commercially available lactoperoxidase from natural source (e.g., ones sold by Biopole), or recombinant lactoperoxidase [e.g., recombinant lactoperoxidase expressed and purified by the method of Shin et al (Biochemical and Biophysical Research Communications), Vol. 271, 2000, p. 831-836] or a commercially available recombinant lactoperoxidase.

Further, as for lactoperoxidase used for the present invention, lactoperoxidase derived from the milk of mammalian is preferable. Lactoperoxidase derived from the milk of bovine, sheep, goat and the like is preferable, and lactoperoxidase derived from the milk of bovine is particularly preferable since these raw materials have been used for long term as food and drink for human being, and therefore a very high level safety thereof to human being has been secured.

In addition, unheated whey derived from bovine milk can be supplied constantly in large scale as a side product of production of dairy products. Therefore, it is particularly preferable as the raw material for preparing the lactoperoxidase according to the present invention.

In the present invention, lactoperoxidase is used as lactoperoxidase system. As for lactoperoxidase system, in general, the combination of the composition which makes a system which catalyzes the production of hypothiocyanate in the presence of lactoperoxidase, hydroperoxidase and thiocyanate, and exhibits a potent antibacterial activity is known.

In general, as for the combination of lactoperoxidase system, for example, the combination of lactoperoxidase, glucose, glucose oxidase and thiocyanate can be mentioned. In the present invention, the antibacterial supplement is used instead of thiocyanate in the lactoperoxidase system. Namely, in the preferred embodiment of the present invention, lactoperoxidase system is the combination of lactoperoxidase, glucose, glucose oxidase and the antibacterial supplement according to the present invention. Further, the composition comprising this combination can be called the lactoperoxidase system antibacterial composition for use according to the present invention in other words.

### [Glucose oxidase]

As for glucose oxidase used for the present invention, for example, a commercially available glucose oxidase (Shinnihon Chemicals Corp. and the like) which is an enzyme produced by microorganisms such as Aspergillus niger, Penicillium chrysogenum can be used.

### [Glucose]

As for glucose used for the present invention, for example, a commercially available glucose (Nihon Shokuhin Kako Co., Ltd. and the like) can be used.

### [Xanthan gum]

As for xanthan gum used for the present invention, for example, a commercially available xanthan gum (San-Ei Gen F. F. I., Inc., CP Kelco and the like) can be used.

These kombu extract, lactoperoxidase, glucose oxidase, glucose and xanthan gum are commercially available as foods or food additives, and thus, can be easily obtained.

### [Antibacterial supplement]

The antibacterial supplement according to the present invention includes as an active ingredient the fraction having a molecular weight of 5000 or less obtained from the kombu extract.

Antibacterial supplement according to the present invention is preferably the extract of one or two or more of kombu selected from the group consisting of makombu (Laminaria japonica), rausukombu (Laminaria diabolica), rishirikombu (Laminaria ochotensis) and hidakakombu (Laminaria angustata).

While the antibacterial supplement according to the present invention can be used in the form of fluid, it can also be dried and used as a powdery antibacterial supplement. Powderizing the antibacterial supplement makes it possible to preserve it for prolonged term and makes the operation during pharmaceutical process easy.

Powderizing the antibacterial supplement can be performed using a standard method. However, it is preferable to eliminate the moisture by freeze-drying or spray drying. Further, it is preferable to mix the salts and dextrin and the like before drying.

The antibacterial supplement according to the present invention can remarkably enhance the antibacterial effect of the conventional antibacterial agent by adding the former to the latter. Further, when the conventional antibacterial agent consists of the combination of multiple ingredients (antibacterial system), the antibacterial supplement according to the present invention can exert the antibacterial effect as one ingredient of the antibacterial agent.

As for the antibacterial supplement according to the present invention, concretely, it is to be added into the lactoperoxidase system antibacterial composition, and thus, it is to be used as one ingredient of the lactoperoxidase system antibacterial composition. Further, the lactoperoxidase system antibacterial composition for use in securing the sanitary conditions in a body of the present invention is preferably a combination of lactoperoxidase, glucose oxidase, glucose and the antibacterial supplement according to the present invention.

The antibacterial supplement according to the present invention also can be used as pharmaceutical composition for enhancing the antibacterial action, agent for enhancing the antibacterial action, and an additive for enhancing the antibacterial action.

In the present invention, "antibacterial" includes all of ideas of inhibiting the proliferation of microorganisms (antibacterial), eliminating the microorganisms and decreasing the same in the objective matter (decontamination), and killing the microorganisms (sterilization).

Therefore, the antibacterial supplement according to the present invention can be called a supplement for eliminating the microorganisms or a supplement for sterilization in other words. In the same way, the antibacterial composition for use according to the present invention can be called a composition for eliminating the microorganisms or a composition for sterilization.

### [Lactoperoxidase system antibacterial composition]

Lactoperoxidase system antibacterial composition indicates a composition comprising lactoperoxidase and an ingredient(s) which exerts antibacterial system of lactoperoxidase system. As for the lactoperoxidase system antibacterial composition, for example, a composition comprising lactoperoxidase, glucose, glucose oxidase and thiocyanate can be mentioned in general. In the lactoperoxidase system antibacterial composition for use according to the present invention, the antibacterial supplement according to the present invention can be used with no addition of thiocyanate or salts thereof.

While the antibacterial supplement according to the present invention can be used as one ingredient of the lactoperoxidase system antibacterial composition, it also can be used for addition to the ordinary lactoperoxidase system antibacterial composition. The antibacterial supplement according to the present invention can remarkably enhance the antibacterial effect of the lactoperoxidase system antibacterial composition, in both case.

When the antibacterial supplement according to the present invention is added into the antibacterial composition, the ratio of the antibacterial supplement to the lactoperoxidase system antibacterial composition (the antibacterial supplement is eliminated when a part of the antibacterial composition is the antibacterial supplement), is preferably 30 to 3000 parts by mass of lactoperoxidase system antibacterial composition per 100 parts by mass of the antibacterial supplement.

The lactoperoxidase system antibacterial composition added with the antibacterial supplement according to the present invention can be called the antibacterial agent according to the present invention in other words.

### [Antibacterial composition]

The antibacterial composition use of the second present invention comprises the antibacterial supplement according to the present invention, lactoperoxidase, glucose oxidase and glucose.

The antibacterial composition for use according to the present invention is effective against Staphylococcus aureus, and therefore, it is disclosed that it can be used for spraying to the place which may cause food poisoning such as a sink of kitchen, a washstand, bathtub and floor of a bathroom, a floor or stool in a lavatory, a veranda and the like where bacteria easily increase.

The antibacterial composition for use according to the present invention has a high level safety because all active ingredients can be taken as foods, and therefore, can be used with no limitation.

The antibacterial composition for use according to the present invention has a high level safety to human being because the active ingredient(s) thereof is (are) one(s) used for food materials such as extract of kombu, milk protein, enzyme and sugars and food additive, and thus, it has a characteristic that the antibacterial composition for use according to the present invention has an antibacterial effect against harmful bacteria such as Staphylococcus aureus by taking it orally and routinely.

The antibacterial composition for use according to the present invention can be a mixture of the antibacterial supplement according to the present invention, lactoperoxidase, glucose oxidase and glucose, or it can also contain other ingredient(s).

For example, the antibacterial composition for use according to the present invention can also contain lactoferrin, lysozyme, immunoglobulin, casein, α-lactalbumin, β-lactoglobulin and the like which are useful proteins present in milk, and lactic acid bacterium and the like which act as probiotics.

Further, in the antibacterial composition for use according to the present invention, any ingredient other than the antibacterial supplement, lactoperoxidase, glucose oxidase and glucose can be optionally selected according to the form of use. For example, they can be optionally used by oral administration, and it is also possible to be fabricated into a tablet, capsule, troche, sirup, granule, powder and the like by using a known method. Further, in addition to administer to human being and animals, the antibacterial composition according to the present disclosure can be broadcasted as a spray or fluid to the place where bacteria easily proliferate and the place the sanitary state of which should be secured, or can be left at rest on the horizontal plane or face of a wall as a solid antibacterial agent.

The antibacterial composition for use according to the present invention can be produced, for example, by preparing, as an active ingredient, the antibacterial supplement, lactoperoxidase, glucose oxidase and glucose with an optional additive(s) such as pharmaceutically acceptable excipient. When the preparation is manufactured, the content of active ingredient in the preparation is usually 0.005 to 20% by mass, preferably 0.05 to 12.5% by mass. When the preparation is manufactured, an additive(s) such as excipient, carrier, binder, disintegrator, lubricant, stabilizer, flavor, diluent, solvent for injection can be used.

As the excipient, for example, sugar derivatives such as lactose, white sugar, glucose, mannitol, sorbitol; starch derivatives such as cornstarch, potatostarch, α-starch, dextrin, carboxymethyl starch; cellulose derivatives such as crystalline cellulose, hydroxypropylcellulose, hydroxypropyl methylcellulose, carboxymethyl cellulose, calcium carboxymethyl cellulose; gum arabic; dextran; pullulan; silicate derivatives such as light anhydrous silicic acid, alumini silicas syntheticus, magnesium aluminometasilicate; phosphate derivatives such as calcium phosphate; carbonate derivatives such as calcium carbonate; sulfate derivatives such as calcium sulfate can be mentioned. As a binder, for example, in addition to above-mentioned excipient, gelatin; polyvinyl pyrrolidone; macrogol and the like can be mentioned. As a disintegrator, for example, in addition to said excipient, chemically modified starch or cellulose derivatives such as sodium croscarmellose, sodium carboxymethyl starch, cross-linked polyvinyl pyrrolidone are mentioned. As a lubricant, for example, talc; stearic acid; metal salts of stearic acid such as calcium stearate, magnesium stearate; colloidal silica; waxes such as Veegum, spermaceti; boric acid; glycol; carboxylic acid such as fumaric acid, adipic acid; sodium carboxylate such as sodium benzoate; sulfate such as sodium sulfate; leucine; lauryl sulfate such as sodium lauryl sulfate, magnesium lauryl sulfate; silicates such as silicic acid anhydride, silicate hydrate; starch derivatives are mentioned. As a stabilizer, for example, esters of parahydroxybenzoate such as methylparaben, propylparaben; alcohols such as chlorobutanol, benzyl alcohol, phenyl ethyl alcohol; benzalkonium chloride; acetic anhydride; sorbic acid and the like are mentioned. As a flavor, for example, a sweetener, acidulant, spice(s) and the like are mentioned. As solvent for injection, for example, water, ethanol, glycerol and the like are mentioned.

Further, the antibacterial composition for use according to the present invention also can be administered after it is formulated in the food and drink. While the dosage and frequency of administration may be varied according to the aimed effect, the method for administration, the duration of therapy, age, body weight and the like, the dosage for an adult can be optionally selected usually from the range of from 10 mg to 10 g, and frequency of administration and the duration of therapy may be optionally selected.

### [Antibacterial composition containing xanthan gum]

The antibacterial composition for use in securing the sanitary conditions in a body of the third present invention is an antibacterial composition containing the antibacterial composition for use according to the second present invention and xanthan gum. The antibacterial composition for use of the third present invention comprises the antibacterial supplement according to the present invention, lactoperoxidase, glucose oxidase, glucose and xanthan gum.

The antibacterial composition can provide thickness, as an agent for providing thickness (thickener), when it is added into the fluid food, fluid medicament and fluid livestock feed, because said composition has thickness by including xanthan gum.

For example, when the antibacterial composition containing xanthan gum is added into a fluid food, the physical property of the fluid food becomes a weak gel state, which achieves the effect that the person with swallowing difficulty (dysphagia) and aged person can swallow with ease.

The antibacterial composition containing xanthan gum can be used preferably for particularly a person with swallowing difficulty (dysphagia) and aged person who have a risk of bacteria infection from the food.

When the fluid food added with the antibacterial composition containing xanthan gum is taken, the fluid having thickness reach every parts in the oral cavity, and thus, it permeates easily between hard tissue of tooth and gingival tissue. Further, residence time in the oral cavity is prolonged, and thus, it has the effect for maintaining and promoting the sanitary of the oral cavity.

Therefore, when the antibacterial composition for use according to the present invention is used in the oral cavity, it is particularly preferred that the antibacterial composition contains xanthan gum.

In the antibacterial composition containing xanthan gum, the ratio of the antibacterial composition other than xanthan gum to xanthan gum is preferably 30 to 3000 parts by mass of xanthan gum per 100 parts by mass of the antibacterial composition other than xanthan gum.

Further, when the antibacterial composition containing xanthan gum according to the present invention is mixed with the fluid food, it is preferable that the antibacterial composition containing xanthan gum is 1.5 to 3.0 parts by mass per 100 parts by mass of the fluid food.

### [Foods and drinks]

The 4th present invention is foods and drinks containing the antibacterial composition for use of the second present invention. The 4th present invention is also foods and drinks containing the antibacterial composition for use of the third present invention.

As for the form of foods and drinks containing the antibacterial composition for use according to the present invention, for example, refreshing drinks, milk-based drinks and the like, or concentrate solution and instant powder of these drinks; dairy products such as processed milk, fermented milk; enteral nutrient foods and instant powder thereof, thickness adjustive drinks, thickness adjustive foods; functional foods and the like are mentioned. Further, drinks such as carbonated drinks, stamina drinks, fruit beverage and the like (including concentrate solution and instant powder of these drinks); frozen dessert such as ice cream, ice sherbet, ice shavings and the like; noodles such as soba, Japanese wheat noodle, strip of bean-jelly, coating of gyoza, coating of Chinese-style steamed meat, Chinese noodle, instant (Chinese) noodles; confectionery such as candy, chewing gum, chocolate, tablet confectionery, munchy, biscuit, jelly, jam, cream, baked goods; processed marine and stock farm products such-as boiled fish paste, ham, sausage and the like; oils and fats and processed foods thereof such as salad oil, tempura oil, margarine, mayonnaise, shortening, whipped cream, dressing; seasoning such as sauce, gravy; soup, stew, salad, daily dish, pickle, bread and the like can be mentioned. Such foods and drinks can be prepared by mixing sugars such as dextrin, starch; proteins such as gelatin, soy protein, corn protein; amino acids such as alanine, glutamine, isoleucine; polysaccharides such as cellulose, gum arabic; oils and fats such as soy oil, medium-chain triglyceride and the like into germicide for oral cavity or food additive (including ones in the form of powder or aqueous solution thereof (sirup)) according to the present invention.

The present invention will be explained with the following working examples. The present invention is not limited to the following working examples.

### EXAMPLE 1

### Preparation of antibacterial supplement according to the present invention

### (1) Preparation of extract of kombu

4 g of commercially available dried makombu (Eight Co-operative Buying Co., Ltd.) was added into 400 ml of purified water in a beaker and soaked in the water for 2 hours at room temperature. Subsequently, the beaker including the makombu was heated at about 95°C for 30 minutes. Then, after the beaker was left for cooling, the content thereof was dezymotized with a filter the pore size of which is 0.45 µm (Advantech Co., Ltd.) and thus, 350 ml of extract of kombu was obtained.

In this example, the extract of kombu was prepared by adding 4 g of kombu sample into 400 ml of purified water, the concentration of which was 2 times one compared to preparing soup stock for cooking.

### (2) Fractionation of extract of kombu

12 ml of extract of kombu was batched off and the fraction having a molecular weight greater than 5000 was eliminated by centrifugation using an ultrafiltration membrane cartridge (Millipore) for 5000 of molecular weight cut off, under the condition of 2000 rpm (960 x g) and 20 minutes, and thus, the permeate (permeated fluid) comprising a fraction having a molecular weight of 5000 or less (corresponding to the antibacterial supplement according to the present invention) was obtained.

### EXAMPLE 2

1 kg of commercially available makombu (Naiya Shouten) was added into 20 L of purified water in a stainless container and soaked in the water for 2 hours at room temperature, and then heated at about 95°C for 30 minutes, and thus, 15 L of extract of kombu was obtained. Subsequently, the extract of kombu was treated with an ultrafiltration membrane module (Asahi Kasei Corporation) for 4000 of molecular weight cut off, and thus, 13 L of permeate comprising a fraction having a molecular weight of 4000 or less was obtained. To this permeate, 450 g of dextrin (Toakasei Co. Ltd.) was added as excipient and dissolved and then freeze-dried, and thus, 500 g of the powdery antibacterial supplement according to the present invention was produced.

In this example, 50 g (dry mass) of the active ingredient of the present invention was obtained from 1 kg of dried makombu. Since 50 g (dry mass) of the active ingredient is included in 500 g (dry mass) of powdery antibacterial supplement according to the present example, the concentration of the active ingredient in said powdery antibacterial supplement is 10 percent by mass.

### EXAMPLE 3

117.6 g of powdery antibacterial composition was obtained by mixing the raw material powders of the antibacterial composition having the following composition. 0.375 g of said powdery antibacterial composition was weighed and added into 250 ml plastic container, and thus, 300 of powdery antibacterial compositions were prepared.

This antibacterial composition contained in the container can be taken as a fluid antibacterial composition by adding 250 ml of water to the powder so that it is dissolved when it is used.

| | |
|---|---|
| The powdery antibacterial supplement prepared in EXAMPLE 2 | 100 g |
| Lactoperoxidase (Biopole) | 0.6 g |
| Glucose oxidase (Shinnihon Chemicals Corp.) | 8.0 g |

| | |
|---|---|
| Glucose (Nihon Shokuhin Kako Co. Ltd.) | 9.0 g |

The action of the antibacterial supplement according to the present invention is explained in detail by indicating the following Test examples.

### [Test example 1]

In this test, it was confirmed that the antibacterial supplement according to the present invention is effective as the antibacterial supplement in the lactoperoxidase system antibacterial composition.

### (1) Preparation of sample

As the sample for test, the permeate of extract of kombu (low molecular weight fraction of extract of kombu) prepared in EXAMPLE 1 was used.

On the other hand, as a control sample, the extract of kombu in EXAMPLE 1 which had not been fractionated was used as it is.

### (2) Preparation of emulsion of the bacteria

Staphylococcus aureus JCM2151 (subdivided from RIKEN, Japan) was overnight cultured in 1% bactopeptone medium. 20 ml of this culture medium was centrifuged using a centrifuge (Hitachi, Ltd.), under condition of 3000 rpm (2150 x g), for 10 minutes, at 4°C, and then, the residue was suspended in 10 ml of phosphate buffered saline, and thus, emulsion of the bacteria was prepared.

### (3) Method of test

Into 5 ml tube, 1.0 ml of the test sample, 0.5 ml of 3.4 % aqueous solution of sodium chloride (Kokusan Chemical Co. Ltd.), 20 µl of 1 mg/ml aqueous solution of lactoperoxidase (Biopole), 30 µl of 1% aqueous solution of glucose (Wako Pure Chemical Industries, Ltd.), 403.3 µl of purified water, and 20 µl of emulsion of the bacteria described in (2) were added and mixed.

Subsequently, 26.7 µl of 10 mg/ml aqueous solution of glucose oxidase (Shinnihon Chemicals Corp.) was added and stirred, and incubated at room temperature for 5 minutes, and then ten-times serial dilutions by volume with phosphate buffered saline were performed. Each 20 µl of the diluted fluids was applied on a standard agar plate and then cultured in the incubator overnight at 37°C, and then, the number of colonies formed on the agar plate were counted. Logarithm number of living bacteria per 1 ml of said test mixed fluid (log₁₀cfu/ml) was calculated from the number of colonies detected.

In the same way, the test was performed using 1.0 ml of the control sample.

Further, with the test sample and control sample, the same tests were performed in the same system as in the above-described test example, except that glucose oxidase was not added.

### (4) The result of the test

The result of the test is shown in Table 1.

In the system where the test sample (low molecular weight fraction of the extract of makombu) was used, the number of living Staphylococcus aureus in the sample mixed fluid was decreased to the level which cannot be detected. On the other hand, in the system where the control sample (the extract of makombu) was used, the number of living Staphylococcus aureus in the sample mixed fluid was almost unchanged. Further, in the system where glucose oxidase was not added, the number of living Staphylococcus aureus was almost unchanged in both of the test sample and control sample.

From this result, it was revealed that the combination of the low molecular weight fraction of the extract of kombu, lactoperoxidase, glucose oxidase and glucose exerts the remarkable antibacterial effect. Further, the extract of kombu does not act as antibacterial agent, but acts as an antibacterial supplement which exerts the antibacterial effect when it is added to the other ingredient.

In the combination using the present test sample, it was surprisingly revealed that the antibacterial effect was exerted in spite of no use of thiocyanate which was previously required at certain concentration or more for action of antibacterial system based on lactoperoxidase system.

Further, it has been revealed that the low molecular weight fraction of the extract of kombu has a remarkable effect, namely it exerts the antibacterial effect as an active ingredient of antibacterial composition with lactoperoxidase system.

Further, it is revealed that while the high molecular weight fraction having a molecular weight greater than 5000 of the extract of kombu has inhibitory substance, said fraction can be used for antibacterial system of lactoperoxidase when the inhibitory substance is eliminated.

**[Table 1]**

| | Number of living Staphylococcus aureus (log₁₀cfu/ml) | |
|---|---|---|
| | glucose oxidase | |
| | added | not added |
| Test sample | N. D. | 5.8 |
| Control sample | 5.4 | 6.2 |

| | | |
|---|---|---|
| N. D. : Not detectable (< 2.70) Initial number of microorganisms: about 6.0 log₁₀cfu/ml | | |

### [Test example 2]

In this test, kinds of kombu which can be used as raw material for antibacterial supplement were searched.

### (1) Preparation of sample

Low molecular weight fractions were prepared using each 4 g of 4 kinds of commercially available dried kombu (makombu, rausukombu, rishirikombu, and hidakakombu), according to the method of EXAMPLE 1.

Sample of each kind of kombu was prepared with three different products obtained from three different companies.

### (2) Preparation of fluid of microorganisms

The fluid of Staphylococcus aureus was prepared in the same way as in Test example 1 (2).

### (3) Method for test

Test was performed in the same way as in Test example 1 (3)

### (4) Result of test

The result of the test is shown in Table 2. The test was performed twice for each sample.

As the result, it was revealed that the low molecular weight fractions of extracts of kombu prepared from all kinds of kombu examined decreased the number of living Staphylococcus aureus to the level which is not detectable, and thus, exhibit a potent antibacterial activity.

From this result, it has been revealed that as the raw materials for the antibacterial supplement according to the present invention, all of makombu, rausukombu, rishirikombu, hidakakombu can be preferably used.

**[Table 2]**

| Kind of kombu | Number of living Staphylococcus aureus(log₁₀cfu/ml) | | |
|---|---|---|---|
| | Sample 1 | Sample 2 | Sample 3 |
| Makombu | N. D. | N. D. | N. D. |
| Rausukombu | N. D. | N. D. | N. D. |
| Rishirikombu | N. D. | N. D. | N.D. |
| Hidakakombu | N. D. | N. D. | N.D. |

| | | | |
|---|---|---|---|
| N. D. : not detectable (< 2.70) Initial number of microorganisms: about 6.0 log₁₀cfu/ml | | | |

### [Test example 3]

The object of this test was to examine the dose dependency of the antibacterial supplement in the antibacterial composition for use according to the present invention.

### (1) Preparation of sample

The sample of the low molecular weight fraction of extract of makombu prepared in EXAMPLE 1 (hereafter, referred to as the antibacterial supplement according to the present invention) was used, the rate of dilution of which was defined as 1. The sample was diluted with purified water to 2 times volume in step-by-step mode, and thus, a twofold dilution series (twofold serial dilutions) ended with 128 times volume dilution was prepared.

### (2) Preparation of fluid of microorganisms

The fluid of Staphylococcus aureus was prepared in the same way as in Test example 1 (2).

### (3) Method of test

Test was performed in the same way as in test example 1, with exception that the antibacterial supplement prepared in EXAMPLE 1 and the twofold dilution series were added into the test mixture.

### (4) Result of test

The result of this test is shown in Table 3.

To 32 times dilution, the antibacterial supplement decreased the number of Staphylococcus aureus to the level which was not still detectable, and even at 64 times dilution, the number was decreased to 1/100 or less of initial number of the microorganisms.

From the result of this test, it has been revealed that the concentration of the antibacterial supplement is correlated with the antibacterial auxiliary effect, and the antibacterial supplement according to the present invention supports the lactoperoxidase system antibacterial action in dose dependent mode.

**[Table 3]**

| Dilution rate of Antibacterial supplement | Number of living Staphylococcus aureus (log₁₀cfu/ml) |
|---|---|
| 1 | N. D. |
| 2 | N. D. |
| 4 | N. D. |
| 8 | N. D. |
| 16 | N. D. |
| 32 | N. D. |
| 64 | 3.4 |
| 128 | 5.6 |

| | |
|---|---|
| N. D. : not detectable (< 2.70) Initial number of microorganisms: about 6.0 log₁₀cfu/ml | |

### [Test example 4]

The object of this test was to confirm that the antibacterial composition containing xanthan gum according to the present invention exerts a remarkable antibacterial activity in spite of addition of xanthan gum.

### (1) Preparation of sample

Antibacterial composition having the following composition was prepared according to the standard method, and antibacterial composition containing xanthan gum (antibacterial composition of the third present invention) was prepared as the product 1 according to the present invention.

| | |
|---|---|
| Powdery antibacterial supplement prepared in EXAMPLE 2 | 10 (%) |
| Lactoperoxidase (Biopole) | 0.06 |
| Glucose oxidase (Shinnihon Chemicals Corp.) | 0.8 |
| Glucose (Nihon Shokuhin Kako Co. Ltd.) | 0.9 |
| Xanthan gum (San-Ei Gen F. F. I., Inc.) | 30 |
| Calcium lactate pentahydrate (Daiichi Kasei Co., Ltd.) | 2.6 |
| Trisodium citrate (San-Ei Gen F. F. I., Inc.) | 2.4 |
| Dextrin (Toakasei Co., Ltd.) | 53.24 |

On the other hand, antibacterial composition having the same formulation with the product according to the present invention excepting that the composition does not contain xanthan gum, as the product 2 according to the present invention. Mass of the product 2 was adjusted so that it would be equal to that of the product 1 except xanthan gum. Further, as a control product 1, a commercially available thickener composition (Morinaga Milk Industry Co., Ltd.) added with none of powdery antibacterial auxiliary (extract of kombu) of EXAMPLE 2, lactoperoxidase, glucose oxidase, and glucose was used.

### (2) Preparation of fluid of organisms

Fluid of Staphylococcus aureus was prepared in the same way as in Test example 1 (2).

### (3) Method for test

Into 50 ml tube, 0.5 g of the antibacterial composition of the present invention described in (1) (product 1 according to the present invention) was added. In the same way, into 50 ml tube, 0.5 g of the control product 1 was added (product 2 according to the present invention). Further, into another 50 ml tube, 0.5 g of a commercially available thickener composition (control) was added. Subsequently, into these tubes, mixed solution of 0.3 ml of said fluid of microorganisms and 30 ml of 0.85% aqueous solution of sodium chloride was added and sufficiently vortexed. After incubation at room temperature for 5 minutes, dilutions with phosphate buffered saline to ten-times volume were performed in step-by-step mode. Each 20 µl of these diluted solution was applied onto the standard agar plate, and cultured overnight in an incubator at 37°C, and then, the number of the colonies formed on the agar plate was counted. From the number of the bacterial colonies detected, logarithm number of living bacteria per 1 ml of said mixed solution (log₁₀cfu/ml) was calculated.

### (4) Result of test

The result of this test is shown in Table 4.

It is clear, from the results using the products 1 and 2 according to the present invention, that the antibacterial composition for use according to the present invention decreased the number of living Staphylococcus aureus to the level which can not be detected, and thus, exhibited a potent antibacterial activity, whether xanthan gum is present or not. On the other hand, the control gave no effect to the number of living Staphylococcus aureus.

Accordingly, it has been revealed that the antibacterial composition containing xanthan gum according to the present invention has a potent antibacterial activity.

The powdery antibacterial supplement used in this test was prepared from the ingredient of the extract of kombu having the molecular weight of 4000 or less. Therefore, it was revealed that as the antibacterial supplement according to the present invention, the ingredient of the extract of kombu having the molecular weight of 4000 or less also can be used.

**[Table 4]**

| Sample | Number of Living Staphylococcus aureus (log₁₀cfu/ml) |
|---|---|
| Product 1 according the present invention | N. D. |
| Product 2 according the present invention | N. D. |
| Control 1 | 6.2 |

| | |
|---|---|
| N. D. : not detectable (< 2.70) Initial number of organisms: about 6.0 log₁₀cfu/ml | |

### INDUSTRIAL APPLICABILITY

The antibacterial composition containing the antibacterial supplement according to the present invention, lactoperoxidase, glucose oxidase and glucose can be used widely as an antibacterial composition having a high safety by using the kombu extract.

Further, the antibacterial composition for use according to the present invention can be used widely as food and drink, medicament, and feed.

## Claims

1. An antibacterial supplement comprising a fraction having a molecular weight of 5000 or less obtained from a kombu extract for use in an antibacterial composition with lactoperoxidase system for securing the sanitary conditions in a body.

2. The antibacterial supplement for use according to claim 1, wherein the kombu extract is an extract of one or more of kombu selected from the group consisting of makombu (Laminaria japonica), rausu kombu (Laminaria diabolica), rishiri kombu (Laminaria ochotensis) and hidaka kombu (Laminaria angustata).

3. An antibacterial composition for use in securing the sanitary conditions in a body comprising the antibacterial supplement according to claim 1 or 2, lactoperoxidase, glucose oxidase and glucose.

4. The antibacterial composition for use according to claim 3, wherein the antibacterial composition further contains xanthan gum.

5. Food and drink containing the antibacterial composition for use according to claim 3 or 4 for securing the sanitary conditions in a body.

6. A method for preparing an antibacterial composition for use in securing the sanitary conditions in a body including as an active ingredient a fraction having a molecular weight of 5000 or less obtained from the kombu extract, lactoperoxidase, glucose oxidase and glucose, comprising
(i) obtaining kombu extract by performing the extraction from the kombu with a solvent(s),
(ii) fractionating the kombu extract into a fraction having a molecular weight of 5000 or less,
(iii) mixing the fractionated fraction having a molecular weight of 5000 or less, lactoperoxidase, glucose oxidase and glucose.

7. The method according to claim 6, wherein the solvent is water or aqueous solution.

8. An additive for use in an antibacterial composition with lactoperoxidase system for securing the sanitary conditions in a body, including a fraction having molecular weight of 5000 or less obtained from a kombu extract, prepared according to the extraction step (i) and fractionation step (ii) as defined in claim 6 or 7.

## Patentansprüche

1. Antibakterielles Supplement, umfassend eine Fraktion mit einem Molekulargewicht von 5.000 oder weniger, erhalten aus einem Kombu-Extrakt, zur Verwendung in einer antibakteriellen Zusammensetzung mit Lactoperoxidasesystem zur Sicherung der sanitären Bedingungen im Körper.

2. Antibakterielles Supplement zur Verwendung gemäß Anspruch 1, wobei der Kombu-Extrakt ein Extrakt von einem oder mehreren Kombu ist, ausgewählt aus der Gruppe, bestehend aus Makombu (Laminaria japonica), Rausu-Kombu (Laminaria diabolica), Rishiri-Kombu (Laminaria ochotensis) und Hidaka-Kombu (Laminaria angustata).

3. Antibakterielle Zusammensetzung zur Verwendung in der Sicherung von sanitären Bedingungen im Körper, umfassend das antibakterielle Supplement gemäß Anspruch 1 oder 2, Lactoperoxidase, Glucoseoxidase und Glucose.

4. Antibakterielle Zusammensetzung zur Verwendung gemäß Anspruch 3, wobei die antibakterielle Zusammensetzung ferner Xanthangummi enthält.

5. Nahrungsmittel und Getränk, enthaltend die antibakterielle Zusammensetzung zur Verwendung gemäß Anspruch 3 oder 4 zur Sicherung der sanitären Bedingungen im Körper.

6. Verfahren zur Herstellung einer antibakteriellen Zusammensetzung zur Verwendung bei der Sicherung der sanitären Bedingungen im Körper, umfassend als aktives Ingrediens eine Fraktion mit einem Molekulargewicht von 5.000 oder weniger, erhalten aus dem Kombu-Extrakt, Lactoperoxidase, Glucoseoxidase und Glucose, umfassend
(i) Erhalten eines Kombu-Extrakts durch Durchführung der Extraktion aus dem Kombu mit einem Lösungsmittel/Lösungsmitteln,
(ii) Fraktionieren des Kombu-Extrakts in eine Fraktion mit einem Molekulargewicht von 5.000 oder weniger,
(iii) Mischen der fraktionierten Fraktion mit einem Molekulargewicht von 5.000 oder weniger, Lactoperoxidase, Glucoseoxidase und Glucose.

7. Verfahren gemäß Anspruch 6, wobei das Lösungsmittel Wasser oder wässrige Lösung ist.

8. Additiv zur Verwendung in einer antibakteriellen Zusammensetzung mit Lactoperoxidasesystem zur Sicherung der sanitären Bedingungen im Körper, umfassend eine Fraktion mit Molekulargewicht von 5.000 oder weniger, erhalten aus einem Kombu-Extrakt, hergestellt gemäß dem Extraktionsschritt (i) und dem Fraktionierungsschritt (ii) wie in Anspruch 6 oder 7 definiert.

## Revendications

1. Supplément antibactérien comprenant une fraction ayant un poids moléculaire de 5000 ou moins, obtenue à partir d'un extrait de kombu pour l'utilisation dans une composition antibactérienne avec le système lactoperoxydase pour assurer les conditions sanitaires dans un corps.

2. Supplément antibactérien pour l'utilisation selon la revendication 1, dans lequel l'extrait de kombu est un extrait d'un kombu ou de plusieurs kombu, choisi(s) parmi le groupe, consistent en makombu (Laminaria japonica), rausu kombu (Laminaria diabolica), rishiri kombu (Laminaria ochotensis) et hidaka kombu (Laminaria angustata).

3. Composition antibactérienne pour l'utilisation pour assurer les conditions sanitaires dans un corps, comprenant le supplément antibactérien selon la revendication 1 ou 2, lactoperoxydase, glucose-oxydase et glucose.

4. Composition antibactérienne pour l'utilisation selon la revendication 3, la composition antibactérienne contenant en outre de la gomme xanthane.

5. Aliment et boisson contenant la composition antibactérienne pour l'utilisation selon la revendication 3 ou 4 pour assurer les conditions sanitaires dans un corps.

6. Procédé de préparation d'une composition antibactérienne pour l'utilisation pour assurer les conditions sanitaires dans un corps, comprenant en tant qu'ingrédient actif une fraction ayant un poids moléculaire de 5000 ou moins, obtenue à partir de l'extrait de kombu, lactoperoxydase, glucoseoxydase et glucose, le procédé comprenant les étapes consistent à
(i) obtenir un extrait de kombu en effectuant l'extraction du kombu avec un solvant (avec des solvants),
(ii) fractionner l'extrait de kombu dans une fraction ayant un poids moléculaire de 5000 ou moins,
(iii) mélanger la fraction fractionnée ayant un poids moléculaire de 5000 ou moins, de la lactoperoxydase, de la glucose-oxydase et du glucose.

7. Procédé selon la revendication 6, dans lequel le solvant est de l'eau ou une solution aqueuse.

8. Additif pour l'utilisation dans une composition antibactérienne avec le système lactoperoxydase pour assurer les conditions sanitaires dans un corps, comprenant une fraction ayant un poids moléculaire de 5000 ou moins, obtenue à partir d'un extrait de kombu, préparé selon l'étape consistant à extraire (i) et l'étape consistant à fractionner (ii) comme défini dans la revendication 6 ou 7.
